(19) Europäisches Patentamt
European Patent Office
Office européen des brevets

(11) Publication number: **0 042 250 B1**

(12) **EUROPEAN PATENT SPECIFICATION**

(45) Date of publication of the patent specification: **07.03.84**

(51) Int. Cl.³: **A 61 K 31/365,** C 07 H 17/08, C 12 P 19/62 // (C12P19/62, C12R1/54)

(21) Application number: **81302571.5**

(22) Date of filing: **10.06.81**

(54) **Macrolide antibiotics.**

(30) Priority: **12.06.80 US 156854**

(43) Date of publication of application: **23.12.81 Bulletin 81/51**

(45) Publication of the grant of the patent: **07.03.84 Bulletin 84/10**

(84) Designated Contracting States: **AT BE CH DE FR GB IT LI LU NL SE**

(56) References cited:
**THE JOURNAL OF ORGANIC CHEMISTRY, vol. 44, no. 12, 8th June 1979, American Chemical Society, pages 2050-2052 A.A. NAGEL et al.: "Selective cleavage of the mycinose sugar from the macrolide antibiotic tylosin: A unique glycosidic scission"**
**CHEMICAL ABSTRACTS, vol. 93, no. 13, 29th September 1980, page 506, no. 130487v Columbus, Ohio, U.S.A. S. OMURA et al.: "Bioconversion and biosynthesis of 16-membered macrolide antibiotics. Part XVI. Isolation and characterization of a new 16-membered lactone, protylonolide, from a mutant of tylosin-producing strain, streptomyces fradiae KA-427"**

(73) Proprietor: **ELI LILLY AND COMPANY, 307, East McCarty Street, Indianapolis Indiana 46285 (US)**

(72) Inventor: **Baltz, Richard Henry, 7446, Sunset Lane, Indianapolis Indiana 46260 (US)**
Inventor: **Wild, Gene Muriel, 7455 Jewel Lane, Indianapolis Indiana 46250 (US)**
Inventor: **Seno,Eugene Thomas, Cottage No. 1 John Innes Institute Colney Lane, Norwich NR4 7UH (GB)**
Inventor: **Kirst, Herbert Andrew, 1819 Madison Village Drive Apt. B-6, Indianapolis Indiana 46227 (US)**

(74) Representative: **Crowther, Terence Roger European Patent Attorney et al, Erl Wood Manor, Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

# Macrolide antibiotics

This invention relates to macrolide antibiotics, in particular to compounds similar to the well-known therapeutic agent tylosin (see, for example, *Tetrahedron Letters*, 2339 (1970)).

Despite tylosin's great value, there exists a constant need to develop new antibiotics, *inter alia*, in view of the possibility of emergence of resistant strains. Further, structural modification can lead no changes in the spectrum of susceptible microorganisms. Unfortunately, chemical modification of the structure of tylosin-like macrolides has proven to be extraordinarily difficult. For instance, in *J. Org. Chem. 44 (12)*, 2050-2 (1979) there is an indication of the problems involved in the cleavage of the mycinosyl glycoside linkage from tylosin by chemical means. Indeed, in the overwhelming majority of cases, research workers in this area, intent on investigating novel structures, have been forced to search for new microorganisms, either naturally-occurring or synthetic, hoping that their cultivation will yield similar structures.

A conversion using microorganisms is described in Japanese Patent application 78/115508 (JP-A-55-43013), which discloses the preparation of a 3-acetyl-4" isovaleryl macrolide from mycaninosyl tylonide and mycarose.

Surprisingly, the Applicants have now discovered that compounds similar to tylosin but lacking tylosin's mycinosyl sugar entity can be prepared by the submerged aerobic fermentation of certain *Streptomyces fradiae* strains.

According to the present invention there is provided a new macrolide antibiotic having the structural formula (I):

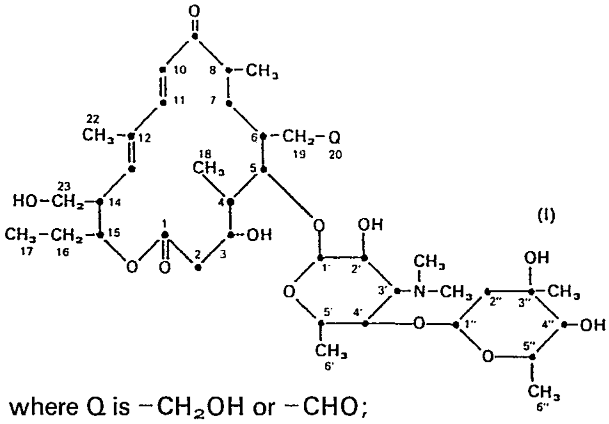

(I)

where Q is $-CH_2OH$ or $-CHO$;

or acyl esters thereof, derived from monocarboxylic acids having from 2 to 18 carbon atoms;

or their acid-addition salts;

provided that when the 3-position of the molecule is occupied by an O-acetyl group, the 4"-position cannot be occupied by an O-isovaleryl group.

Although no stereochemical configuration is indicated for the structure above, it is to be understood that the stereochemical configuration is identical to that of tylosin. The neutral sugar is mycarose and the amino-sugar is mycaminose.

When Q is $-CHO$, the compound of formula (I) is the new macrolide antibiotic: 23-Demycinosyltylosin which will be called demycinosyltylosin or DMT for convenience herein and which has the structure:

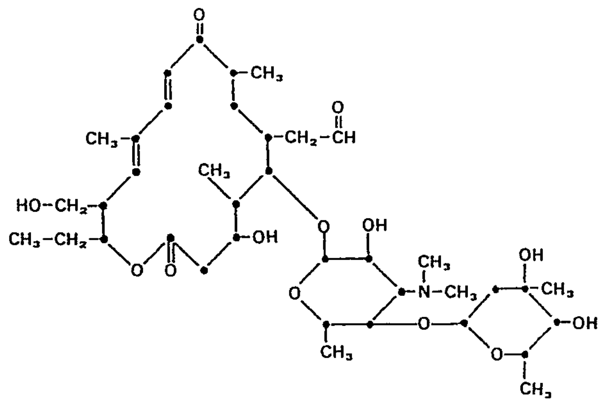

The dihydro-derivative of DMT, i.e. 20-dihydro-23-demycinosyltylosin, will be called dihydro-DMT for convenience herein, and has the structure:

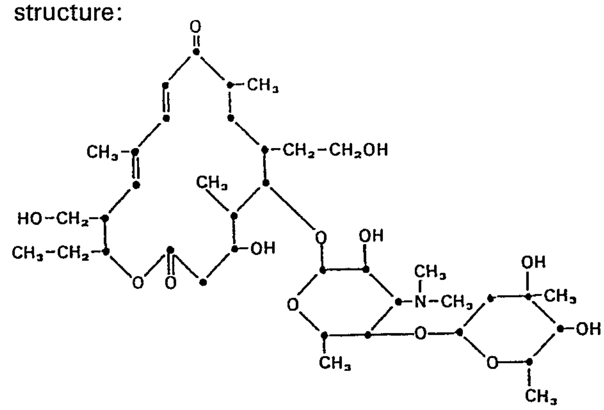

The compounds of the invention inhibit the growth of microorganisms which are pathogenic to warmblooded animals. More particularly, they are antibacterial agents which are especially active against gram-positive microorganisms and *Mycoplasma* species.

The hydroxyl groups of DMT and dihydro-DMT can be esterified on the 2', 4", 3", 23 and 3-hydroxyl groups to form useful acyl ester derivatives. In addition, dihydro-DMT can be esterified on the 20-hydroxyl group. Esterification of the 2'-hydroxyl group is most facile.

DMT, dihydro-DMT and their acyl ester derivatives are basic compounds which, when treated with acids, are converted to acid addition salts. These addition salts also form part of this invention. To simplify discussions of utility, the term «DMT compound» will be used hereinafter and refers to DMT, dihydro-DMT, a specified acyl ester derivative of these compounds, or a pharmaceutically acceptable acid addition salt of DMT, dihydro-DMT or of their acyl ester derivatives.

This invention further relates to a new strain of *Streptomyces fradiae*, NRRL 12170, and to the

method of producing DMT or dihydro-DMT by culturing this strain under submerged aerobic fermentation conditions until a substantial level of antibiotic activity is produced. DMT or dihydro-DMT can be extracted from basified broth filtrate with polar organic solvents, and can be further purified by extraction, adsorption and/or crystallization.

This invention also relates to improved methods of preparing 5-O-mycaminosyltylonolide (OMT) and 20-dihydro-5-O-mycaminosyltylonolide (dihydro-OMT) by mild acid hydrolysis of DMT and dihydro-DMT, respectively.

The dihydro derivative of DMT can be obtained by chemical reduction or by fermentation. When preparing dihydro-DMT by chemical reduction, known procedures such as, for example, treating DMT with an approximately stoichiometric amount of a chemical reducing agent such as sodium borohydride in for instance an alcoholic solvent, may be used. Dihydro-DMT can also be produced by the *S. fradiae* NRRL 12170 of this invention under controlled fermentation conditions.

DMT and dihydro-DMT can be esterified at the 2′, 4″, 3″, 23 and 3-positions to give acyl ester derivatives by treatment with acylating agents using methods known in the art. In addition, dihydro-DMT can be esterified at the 20-position. Esterification of the 2′-hydroxyl group is most facile. Typical acylating agents include anhydrides, halides (usually in combination with a base or other acid scavenger) and active esters of organic acids. Acylation can also be achieved by using a mixture of an organic acid and a dehydrating agent such as N,N′-dicyclohexylcarbodiimide. Acylations can also be carried out enzymatically as described by Okamoto et al. in U.S. Patent No. 4,092,473. Once formed, the acyl derivatives can be separated and purified by known techniques.

The 2′-monoester derivatives can be prepared by selective esterification techniques generally known in the art, such as, for example, treatment of the antibiotic with a stoichiometric quantity (or a slight excess) of an acylating agent, such as an acyl anhydride, at about room temperature for from about 1 to about 24 hours until esterification is substantially complete. The 2′-monoester can be isolated from the reaction mixture by standard procedures such as extraction and chromatography and crystallization.

Useful acyl esters are those of organic acids including aliphatic, cycloaliphatic, aryl, aralkyl, or heterocyclic carboxylic acids having from 2 to 18 carbon atoms, most preferably from 2 to 12 carbon atoms.

Representative suitable esters include those derived from acids such as acetic, chloroacetic, propionic, butyric, isovaleric, alkoxycarbonic, stearic, cyclopropanecarboxylic, cyclohexanecarboxylic, β-cyclohexylpropionic, 1-adamantanecarboxylic, benzoic, phenylacetic, phenoxyacetic, mandelic and 2-thienylacetic acids, the aryl- and aralkylacids optionally bearing substituents such as halogen, nitro, lower alkoxy and the like on the aromatic moiety.

Pharmaceutically acceptable ester derivatives are a preferred group. Other ester derivatives are useful, however, as intermediates.

DMT, dihydro-DMT and their specified acyl ester derivatives form acid addition salts. The acid addition salts of DMT, dihydro-DMT and of their acyl derivatives also form part of this invention. Such salts are useful, for example, for separating, purifying and crystallizing DMT, dihydro-DMT and their acyl derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, *d*-camphoric, glutaric, glycolic, phthalic, tartaric, formic, lauric, stearic, salicyclic, methanesulfonic, benzenesulfonic, sorbic, picric, benzoic, cinnamic and the like acids.

Pharmaceutically acceptable acid addition salts are an especially preferred group of salts of this invention. «Pharmaceutically acceptable» salts are salts which are sufficiently non-toxic to be usable in the chemotherapy of bacterial infections in warmblooded animals.

This invention also relates to improved methods of preparing OMT and dihydro-OMT by mild acid hydrolysis of DMT and dihydro-DMT, respectively. OMT and dihydro-OMT have the same structures as DMT and dihydro-DMT except that the mycarosyl group is replaced by hydrogen. Mild acid hydrolysis conditions are known in the art. Appropriate solutions having a pH of about four or below can be used to accomplish the hydrolysis. Temperatures of about 20° to about 100°C can be used in this method. The reaction time needed to carry out the hydrolysis varies, depending upon the pH of the reaction mixture and the temperature used. At higher pH levels the reaction rate is slower, and at higher temperatures the reaction rate is faster. The reaction is carried out by treating either DMT or dihydro-DMT with a mild acid solution for a time sufficient to effect removal of the mycarosyl group to give OMT or dihydro-OMT, respectively.

Alternatively, and sometimes preferably, OMT or dihydro-OMT can be prepared by treating DMT or dihydro-DMT in the fermentation broth in which it is produced, using mild acidic conditions as above described for a time sufficient to convert the DMT or dihydro-DMT to OMT or dihydro-OMT, respectively. OMT or dihydro-OMT thus prepared can be isolated from the fermentation broth using techniques known in the art.

OMT and dihydro-OMT are described by Gorman et al. in U.S. Patent No. 3,459,853. In U.S. Patent No. 3,459,853 OMT and dihydro-OMT are prepared by controlled acid hydrolysis of tylosin, desmycosin, macrocin, and lactenocin and their dihydro-derivatives. The art method of preparation involves removal of all the neutral sugars from tylosin, desmycosin, macrocin, or lactenocin to

obtain OMT. Analogous methods are taught to prepare dihydro-OMT. Removal of the neutral sugar at the 23-position requires conditions which result in marked product losses. The process of this invention avoids such losses.

As noted previously, DMT and dihydro-DMT can be prepared by culturing a strain of *Streptomyces fradiae* which produces these compounds under submerged aerobic conditions in a suitable culture medium until substantial antibiotic activity is produced. As will be appreciated by those skilled in the art, DMT is produced first in the fermentation process. Dihydro-DMT is produced when the fermentation is carried out for a longer time, thus permitting the DMT present to be reduced enzymatically.

The culture medium used to grow *Streptomyces fradiae* NRRL 12170 can be any one of a number of media. For economy in production, optimal yield, and ease of product isolation, however, certain culture media are preferred. Thus, for example, preferred carbon sources in large-scale fermentation include carbohydrates such as dextrin, glucose, starch, and corn meal, and oils such as soybean oil. Preferred nitrogen sources include corn meal, soybean meal, fish meal, amino acids and the like. Among the nutrient inorganic salts which can be incorporated in the culture media are the customary soluble salts capable of yielding iron, potassium, sodium, magnesium, calcium, ammonium, chloride, carbonate, sulfate, nitrate, and like ions.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/l) of an antifoam agent such as polypropylene glycol (M.W. about 2000) to large-scale fermentation media if foaming becomes a problem.

For production of substantial quantities of DMT or dihydro-DMT, submerged aerobic fermentation in tanks is preferred. Small quantities of DMT or dihydro-DMT may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank. The medium used for the vegetative inoculum can be the same as that used for larger fermentations, but other media can also be used.

*S. fradiae* NRRL 12170 can be grown at temperatures between about 10° and about 37°C. Optimum antibiotic production appears to occur at temperatures of about 28°C.

As is customary in aerobic submerged culture processes, sterile air is bubbled through the culture medium. For efficient antibiotic production the percent of air saturation for tank production should be about 30% or above (at 28°C and one atmosphere of pressure).

Antibiotic production can be followed during the fermentation by testing samples of the broth against organisms known to be sensitive to these antibiotics. One useful assay organism is *Staphylococcus aureus* ATCC 9144. The bioassay is conveniently performed by an automated turbidometric method. In addition, antibiotic production can be readily monitored by high-performance liquid chromatography with UV detection.

Following its production under submerged aerobic fermentation conditions, DMT or dihydro-DMT can be recovered from the fermentation medium by methods used in the fermentation art. Recovery of DMT or dihydro-DMT is accomplished by an initial filtration of the fermentation broth. The filtered broth can then be further purified to give the desired antibiotic. A variety of techniques may be used in this purification. A preferred technique for purification of the filtered broth involves adjusting the broth to about pH 9; extracting the broth with a suitable solvent such as ethyl acetate, amyl acetate, or methyl isobutyl ketone; extracting the organic phase with an aqueous acidic solution; and precipitating the antibiotic by making the aqueous extract basic. Further purification involves the use of extraction, adsorption and/or precipitation techniques.

The new microorganism of this invention was obtained by chemical mutagenesis of a *Streptomyces fradiae* strain which produced tylosin. The microorganism obtained by mutagenesis produces only minimal amounts of tylosin, but produces DMT as a major component.

For characterization purposes, the new organism was compared with *Streptomyces fradiae* strain M48-E2724.1, a tylosin-producing strain derived from *S. fradiae* NRRL 2702. *S. fradiae* NRRL 2702 was disclosed by Hamill et al. in U.S. Patent No. 3,178,341. In the discussions herein the tylosin-producing *S. fradiae* M48-E2724.1 culture will be called «E2724.1».

The new DMT- and dihydro-DMT-producing strain NRRL 12170 is also classified as a strain of *Streptomyces fradiae*. In characterizing this organism, the methods recommended for the International *Streptomyces* Project for the characterization of *Streptomyces* species have been followed [E. B. Shirling and D. Gottlieb, «Methods For Characterization of *Streptomyces* Species,» *Internal. Journal of Systematic Bacteriology, 16* (3), 313-340 (1966)] along with certain supplementary tests. The following references to *S. fradiae* in the literature were consulted: 1) R. E. Buchanan and N. E. Gibbons, «Bergey's Manual of Determinative Bacteriology,» 8th ed., The Williams and Wilkins Co., Baltimore, Md., 1974, p. 815; and 2) E. B. Shirling and D. Gottlieb, «Cooperative Description of *Streptomyces*. II. Species Description from First Study,» *Internal. Journal of Systematic Bacteriology, 18* (2), 118, (1968).

The following description of the DMT-producing strain compares its characteristics with those of the tylosin-producing *S. fradiae* strain «E2724.1».

The spore-chain morphology of the new strain and of the E2724.1 strain is in the Retinaculum-Apertum (RA) section. Hooks, loops, and irregular coils are short and generally not of a wide diameter. This is best observed on ISP#2 (yeast-malt extract agar). The spore surface is smooth; the spore shape is spherical with an average size of 0.65 μM in diameter. The diameter range is from 0.61 to 0.71 μM.

The most obvious differences between these strains are seen in their cultural characteristics. The E2724.1 strain produces aerial mycelia fairly well on most media and is in the White color series. The DMT-producing strain produces very little if any aerial mycelia. When present, it is in the White to Gray color series. The reverse sides of these colonies have no distinctive pigments produced. They are light to moderate yellow in color. Melanoid pigment production is negative[1].

A summary of the important similarities and differences between the E2724.1 strain and the DMT-producing strain is given in Table 1. →

→ *Table 1*

Comparison of *Streptomyces fradiae* E2724.1 and NRRL 12170

| Similarities | Differences |
|---|---|
| Spore-chain morphology | Cultural characteristics |
| Spore-surface ornamentation | Carbon utilization |
| Spore size | Gelatin utilization |
| Lack of chromogeneicity | NaCl tolerance |
| Lack of soluble pigments | pH range |
| Growth in selected vegetative media | Temperature range |
| Starch hydrolysis | |
| Negative skim milk reaction | |
| Nitrate reduction | |
| Catalase positive | |
| Phosphatase positive | |
| Urease negative | |
| Antibiotic sensitivity pattern | |

The morphology and growth characteristics of the *S. fradiae* E2724.1 and NRRL 12170 strains are compared in Table 2. In the tables which follow the antibiotic sensitivities (Table 3), carbon utilization (Table 4) and miscellaneous physiological characteristics (Table 5) are compared.

*Table 2*

Growth Characteristics and Morphology

| | | E2724.1 | NRRL 12170 |
|---|---|---|---|
| Sporophores | | RA | RA |
| Spore chains | | >10 | >10 |
| Spore surface[1] | | smooth | smooth |
| Spore shape | | spherical | spherical |
| ISP#2 | G[2] | good | good |
| | R | 87. m. yellow[3] | 87. m. yellow |
| | Am | good 263. white | fair 263. white |
| | Sp | none | none |
| ISP#3 | G | poor | poor |
| | R | 263. white | 263. white |
| | Am | poor 263. white | trace |
| | Sp | none | none |
| ISP#4 | G | abundant | good |
| | R | 87. m. yellow | 87. m. yellow |
| | Am | abundant 263. white | poor |
| | Sp | none | none |
| ISP#5 | G | good | good |
| | R | 86.1. yellow | 86.1. yellow |
| | Am | good 92. y. white | none |
| | Sp | none | none |
| ISP#7 | G | abundant | good |
| | R | 87. m. yellow | 87. m. yellow |
| | Am | abundant 263. white | fair 265. med. gray |
| | Sp | none | light brown |

[1] Melanoid-pigment production was tested using ISP#1 (tryptone-yeast extract broth), ISP#6 (peptone yeast extract-iron agar), ISP#7 (tyrosine agar), and ISP#7 agar without tyrosine.

*Table 2* (continued)

Growth Characteristics and Morphology

| | | E2724.1 | NRRL 12170 |
|---|---|---|---|
| Bennett's | G | poor | no growth |
| | R | 90. gy. yellow | — |
| | Am | none | — |
| | Sp | none | — |
| Ca-malate | G | good | poor |
| | R | 263. white | 92. y. white |
| | Am | good 263. white | none |
| | Sp | none | none |
| Czapek's | G | good | fair |
| | R | 87. m. yellow | 87. m. yellow |
| | Am | abundant 263. white | trace |
| | Sp | none | none |
| Glucoseasparagine | G | no growth | no growth |
| | R | — | — |
| | Am | — | — |
| | Sp | — | — |
| Tomato paste-oatmeal | G | abundant | good |
| | R | 92. y. white | 87. m. yellow |
| | Am | abundant 263. white | none |
| | Sp | none | none |

[1] Spore-surface ornamentation was determined using a scanning electron microscope.
[2] G = Growth; R = Reverse or underside of colony; Am = Aerial mycelium; Sp = Soluble pigment.
[3] Color names were assigned using the ISCC-NBS color charts (K. L. Kelly and D. B. Judd, "The ISCC-NBS Centroid Color Charts Standard Sample No. 2106," U.S. Dept. of Commerce, National Bureau of Standards, Washington, D.C. 20234).

*Table 3*

Antibiotic Sensitivity[a, b]

| Antibiotics | Conc. | Class Compound | E2724.1 | NRRL 12170 |
|---|---|---|---|---|
| Chloramphenicol | 30 μg | Nitrophenyl compound | + | + |
| Erythromycin | 15 μg | Macrolide | tr | tr |
| Cephaloridine | 30 μg | β-Lactam | + | + |
| Lincomycin | 2 μg | Lincosaminide | – | – |
| Polymyxin B | 300 units | Peptide | tr | tr |
| Streptomycin | 10 μg | Aminoglycoside | + | + |
| Tetracycline | 30 μg | Tetracycline | + | + |
| Vancomycin | 30 μg | Glycopeptide | + | + |

[a] Determined by using sensitivity discs padded onto seeded-agar plates.
[b] – = resistance (no zones of inhibition),
\+ = sensitivity (zones of inhibition),
tr = trace of sensitivity.

*Table 4*

Carbon Utilization[a, b]

| Carbon Source | E2724.1 | NRRL 12170 |
|---|---|---|
| Control: no carbon | – | – |
| Control: glucose | + | + |
| L-Arabinose | – | + |
| D-Fructose | + | + |
| D-Galactose | + | + |

*Table 4* (continued)

Carbon Utilization[a, b]

| Carbon Source | E2724.1 | NRRL 12170 |
|---|---|---|
| i-Inositol | + | + |
| D-Mannitol | − | + |
| Raffinose | − | − |
| Salicin | − | − |
| Sucrose | + | + |
| D-Xylose | + | + |
| D-Rhamnose | − | + |

[a] − = no utilization,
+ = utilization.

[b] Determined on International *Streptomyces* Project (ISP) #9 (carbon-utilization agar) basal medium to which filter-sterilized carbon sources were added to equal a final concentration of 1.0%. Plates were incubated at 30°C and observed after 7 and 12 days.

*Table 5*

Miscellaneous Physiological Characteristics

| | E2724.1 | NRRL 12170 |
|---|---|---|
| ISP#1 (chromogenicity) | − | − |
| ISP#6 (chromogenicity) | − | − |
| ISP#7 (chromogenicity) | − | − |
| Gelatin liquefaction | − | + |
| Skim-milk reaction | − | − |
| pH growth range[1, 2] | 6.1-8.8 | 6.1-7.8 |
| Temperature growth range[1, 3] | 10-37°C | 10-30°C |
| NaCl tolerance[1, 4] | 8% | 4% |
| Starch hydrolysis[5] | + | + |
| Nitrate reduction | + | + |
| Catalase[6] | + | + |
| Phosphatase[6] | + | + |
| Urease[6] | − | − |

[1] On ISP#2 (yeast extract-malt extract agar) medium; incubated 7 days.

[2] Determined using the following buffers at a concentration of 0.05 M: citric acid, pH 3, 4, 5; 2-(N-morpholino)ethanesulfonic acid, pH 6; 3-(N-morpholino)propanesulfonic acid, pH 7; N-2-hydroxyethylpiperazine-N'-2-ethanesulfonic acid, pH 8; 2-amino-2-(hydroxymethyl)-1,3-propanediol, pH 9; 3-cyclohexylamino-1,1-propanesulfonic acid, pH 10, 11. The pH of the agar after seven days' incubation was taken as the correct value since some of the buffers failed to hold their adjusted pH. Buffer toxicity was tested by adjusting all the buffers to pH 7.0 and determining growth. No toxicity was noted.

[3] Tested at 5, 10, 15, 20, 25, 30, 37, 40, 45, 50 and 55°C.

[4] Measured by adding NaCl to the agar to equal: 0, 2, 4, 6, 8, 10 and 12% NaCl by weight.

[5] Starch hydrolysis was determined by testing for the presence of starch with iodine on ISP#4 (inorganic salts-starch agar) plates.

[6] The methods of Blazevic and Ederer were followed for the enzyme assays (D. J. Blazevic and G. M. Ederer, "Principles of Biochemical Tests in Diagnostic Microbiology," John Wiley and Sons, New York, N.Y., 1975).

Based on the foregoing characteristics the DMT- and dihydro-DMT-producing organism, NRRL 12170, is classified as a new strain of *Streptomyces fradiae*. This culture has been deposited and made part of the stock culture collection of the Northern Regional Research Center, Agricultural Research, North Central Region, 1815 North University Street, Peoria, Illinois, 61604, from which it is available to the public under the accession number NRRL 12170.

As is the case with other organisms, the characteristics of *Streptomyces fradiae* NRRL 12170 are subject to variation. For example, artificial variants and mutants of the NRRL 12170 strain may be obtained by treatment with various known physical and chemical mutagens, such as ultraviolet rays, X-rays, gamma rays, and N-methyl-N'-nitro-N-nitrosoguanidine. All natural and artificial variants, mutants and recombinants of *Streptomyces fradiae* NRRL 12170 which retain the characteristic of production of DMT are encompassed in this invention.

The DMT compounds inhibit the growth of pathogenic bacteria, especially gram-positive bacteria and *Mycoplasma* species. Table 6 summarizes the minimal inhibitory concentrations (MIC), as measured by standard agar-dilution assays, at which DMT (free base) inhibits certain bacteria.

*Table 6*

*In Vitro* Activity of DMT Free Base

| Organism | MIC (μg/ml) |
|---|---|
| *Streptococcus pyogenes* C203 | 0.25 |
| *Streptococcus pneumoniae* Park I | 0.13 |
| *Streptococcus* sp. (Group D) 282 | 0.5 |
| *Staphylococcus aureus* 3055 | 1.0 |
| *Staphylococcus aureus* 209P | 0.25 |
| *Pasteurella multocida* | 3.12 |
| *Pasteurella hemolytica* | 12.5 |
| *Mycoplasma gallisepticum* | 0.097 |
| *Mycoplasma hyopneumoniae* | 0.195 |
| *Mycoplasma hyorhinis* | 0.78 |

The DMT compounds have shown *in vivo* antimicrobial activity against experimental bacterial infections. When two doses of test compound were administered to mice in experimental infections, the activity observed was measured as an $ED_{50}$ value [effective dose in mg/kg to protect 50% of the test animals: see Warren Wick, et al., *J. Bacteriol. 81*, 233-235 (1961)]. The $ED_{50}$ values observed for DMT and DMT tartrate are given in Table 7.

*Table 7*

Subcutaneous and Oral $ED_{50}$ Values (mg/kg × 2)

| Compound | *Streptococcus pyogenes* C203 | | *Streptococcus pneumoniae* Park I | | *Staphylococcus aureus* 3055 | |
|---|---|---|---|---|---|---|
| | Subcut. | Oral | Subcut. | Oral | Subcut. | Oral |
| DMT Base | 2.2 | 218 | 15.7 | 66 | 2.7 | 64 |
| DMT Tartrate | 2.3 | 172 | ≤9.9 | 100 | NT* | NT |
| Bacterial Challenge (× $LD_{50}$) | 13 | 11.3 | 3 | 2.7 | 21 | 26 |

* Not tested.

Activity against *Mycoplasma* species is an especially useful aspect of the DMT compounds. Tables 8 and 9 summarize experiments using DMT and DMT tartrate for the treatment of induced *Mycoplasma gallisepticum* infections in chickens. The compounds were administered in the drinking water (at 1 and 2 g/gal for 1 to 3 days) or by injection at 15 or 30 mg/kg of body weight.

*Table 8*

Treatment of induced *Mycoplasma gallisepticum* infection in chicks with DMT base

| Compound | Dosage | No. Died/Total No. | No. with Air Sac Lesions/Total No. | Avg. Wt. (g) | No. with *M. gallisepticum* Antibody/Total No. |
|---|---|---|---|---|---|
| DMT Base | 2.0 g/gal; 1-3 days | 9/30 (30%) | 26/30 (86.7%) | 397 | 21/21 (100%) |
| DMT Base | 1.0 g/gal; 1-3 days | 13/30 (43.3%) | 28/30 (93.3%) | 392 | 15/17 (88.2%) |
| DMT Base | 15 mg/kg | 10/30 (33.3%) | 24/30 (80%) | 453 | 20/20 (100%) |
| Infected Control | — | 19/30 (63%) | 30/30 (100%) | 304 | 11/11 (100%) |

*Table 9*

Treatment of induced *Mycoplasma gallisepticum* infection in chicks with DMT tartrate

| Compound | Dosage | No. Died/Total No. | No. with Air Sac Lesions/Total No. | Avg. Wt. (g) | No. with *M. gallisepticum* Antibody/Total No. |
|---|---|---|---|---|---|
| DMT Tartrate | 2.0 g/gal; 1-3 days | 0/30 (0%) | 13/30 (43.3%) | 458 | 17/30 (56.7%) |
| DMT Tartrate | 1.0 g/gal; 1-3 days | 2/30 (6.7%) | 22/29 (75.9%) | 354 | 27/28 (96.4%) |
| DMT Tartrate | 30 mg/kg | 10/30 (33.3%) | 27/30 (90%) | 330 | 16/20 (80%) |
| Infected Control | — | 15/30 (50%) | 30/30 (100%) | 231 | 15/15 (100%) |

For the prevention or treatment of *Mycoplasma* infections in poultry, a non-toxic amount of a DMT compound is administered to birds orally or parenterally. DMT compounds are most conveniently administered with a pharmaceutically acceptable carrier, such as the water ingested by the birds.

Studies on the acute toxicity of DMT are summarized in Table 10. In these studies, 4- to 5-week-old Harlan ICR mice were used. The median lethal dose ($LD_{50}$) was determined for DMT by the oral (p.o.), subcutaneous (s.c.), intravenous (i.v.), and intraperitoneal (i.p.) routes of administration.

*Table 10*

Acute Toxicity of DMT

| Route | $LD_{50}$ (mg/kg) | |
|---|---|---|
| | Males | Females |
| p.o. | — | >5000 |
| s.c. | — | 5447 |
| i.v. | 100 | 186 |
| i.p. | 721 | 325 |

Clearly, when treating diseases in warmblooded animals, it will usually be the case that the compounds of the invention will be administered in the form of veterinary formulations. Accordingly, in one aspect of the invention there is provided a veterinary formulation which comprises as an active ingredient a compound af formula (I), or a pharmaceutically-acceptable salt or ester thereof, associated with at least one suitable inert carrier therefor.

Those skilled in the art will readily appreciate the nature of carriers of use in such formulations, these carriers being similar to those already utilized in respect of tylosin.

In order to illustrate more fully the operation of this invention, the following Examples are provided:

*Example 1:*

A. *Shake-flask Fermentation of DMT*

A lyophilized pellet of *Streptomyces fradiae* NRRL 12170 was dispersed in 1-2 ml of sterilized water. A portion of this solution (0.5 ml) was used to inoculate a vegetative medium (150 ml) having the following composition:

| Ingredient | Amount (%) |
|---|---|
| Corn steep liquor | 1.0 |
| Yeast extract | 0.5 |
| Soybean grits | 0.5 |
| $CaCO_3$ | 0.3 |
| Soybean oil (crude) | 0.45 |
| Deionized water | 97.25 |

Alternatively, a vegetative culture of *S. fradiae* NRRL 12170 preserved, in 1 ml volumes, in liquid nitrogen was rapidly thawed and used to inoculate the vegetative medium. The inoculated vegetative medium was incubated in a 500 ml Erlenmeyer flask at 29°C for about 48 hours on a closed-box shaker at 300 rpm.

This incubated vegetative medium (0.5 ml) was used to inoculate 7 ml of a production medium having the following composition:

| Ingredient | Amount (%) |
|---|---|
| Beet molasses | 2.0 |
| Corn meal | 1.5 |
| Fish meal | 0.9 |
| Corn gluten | 0.9 |
| NaCl | 0.1 |
| $(NH_4)_2HPO_4$ | 0.04 |
| $CaCO_3$ | 0.2 |
| Soybean oil (crude) | 3.0 |
| Deionized water | 91.36 |

The inoculated fermentation medium was incubated in a 50 ml bottle at 29°C for about 6 days on a closed-box shaker at 300 rpm.

B. *Tank Fermentation of DMT*

In order to provide a larger volume of inoculum, 1200 ml of incubated vegetative medium, prepared in a manner similar to that described in section A, was used to inoculate 250 gallons of a second-stage vegetative growth medium having the following composition:

| Ingredient | Amount (%) |
|---|---|
| Corn steep liquor | 1.0 |
| Soybean oil meal | 0.5 |
| Yeast extract | 0.5 |
| $CaCO_3$ | 0.3 |
| Soybean oil (crude) | 0.5 |
| Lecithin (crude) | 0.015 |
| Water | 97.185 |

The pH was adjusted to 8.5 with 50% NaOH solution.

This second-stage vegetative medium was incubated in a 350-gallon tank for about 48 hours at 28° C, with adequate aeration and agitation.

Incubated second-stage medium (144 gallons) thus prepared was used to inoculate 1000 gallons of sterile production medium having the following composition:

| Ingredient | Amount (%) |
|---|---|
| Fish meal | 0.875 |
| Corn meal | 1.5 |
| Corn gluten | 0.875 |
| $CaCO_3$ | 0.2 |
| NaCl | 0.1 |
| $(NH_4)_2HPO_4$ | 0.04 |
| Beet molasses | 2.0 |
| Soybean oil (crude) | 3.0 |
| Lecithin | 0.09 |
| Water | 91.32 |

The pH was adjusted to 7.2 with 50% NaOH solution.

The inoculated production medium was allowed to ferment in a 1600-gallon tank for 8 to 9 days at a temperature of 28°C. The fermentation medium was aerated with sterile air to keep the dissolved oxygen level between about 30% and 50% and was stirred with conventional agitators at about 250 rpm.

*Example 2:*

*Isolation of DMT*

Harvested whole broth (3800 l), obtained as described in Example 1, was filtered, using a filter aid. The mycelial cake was washed with water, and this water wash added to the filtrate.

The pH of the filtrate was adjusted to pH 9.2, using a 50% aqueous solution of sodium hydroxide (9.5 l), the filtrate then being extracted with ethyl acetate (2000 l). Deionized water (450 l) and sodium phosphate monobasic (6.4 kg) were added to the ethyl acetate extract and the resulting solution thoroughly mixed. The pH of this solution was adjusted from about pH 6.0 to pH 4.35, using a phosphoric acid solution (3300 ml; 2 parts water to one part phosphoric acid). The aqueous phase was separated. The pH of the enriched aqueous phase was adjusted to pH 6.5 using a 50% aqueous sodium hydroxide solution (700 ml).

The resulting solution was concentrated to a volume of about 225 l under vacuum. The pH of the concentrated solution was adjusted to pH 9.2 by the addition of 10% aqueous sodium hydroxide (16 l). The resulting basic solution was permitted to stand overnight. The crystals which formed were separated by filtration, washed with deionized water (50 l), and dried to give about 8.6 kg of product. A portion of the product thus obtained (3 kg) was recrystallized from acetone-water to give about 2.07 kg of DMT free base.

DMT softens at about 132° and slowly melts up to about 150°C. Elemental analysis of DMT indicates that it has the following approximate percentage composition: carbon, 61%; hydrogen, 8.5%; nitrogen, 2%; oxygen, 28%. DMT has an empirical formula of $C_{38}H_{63}NO_{13}$ and a molecular weight of about 742 (741 as determined by mass spectrometry).

The infrared absorption spectrum of DMT free base in chloroform is shown in the accompanying drawing. Observable absorption maxima occur at the following frequencies ($cm^{-1}$): 3634 (v. small), 3559 (shoulder), 3423 (broad), 2955 (intense), 2907 (intense), 1710 (intense), 1676 (shoulder), 1588 (intense), 1447 (shoulder), 1396 (shoulder), 1359 (small), 1309 (v. small), 1178 (shoulder), 1156 (intense), 1111 (shoulder), 1072 (shoulder), 1048 (intense), 1013 (shoulder), 984 (shoulder), 926 (v. small), 898 (v. small), and 833 (v. small).

The ultraviolet absorption spectrum of DMT in neutral ethanol exhibits an absorption maximum at 283 nm (ε 22,296; $E^{1\%}_{1\,cm}$ = 300.9).

DMT (free base) has the following specific rotation: $[\alpha]^{25}_{D}$ −53.5° (c 1, $CH_3OH$).

Electrometric titration of DMT in 66% aqueous dimethylformamide indicates the presence of a titratable group with a $pK_a$ value of about 7.25.

DMT base is soluble in water and in most polar organic solvents, such as acetone, methanol, ethanol, chloroform, dimethylformamide and dimethyl sulfoxide. DMT acid addition salts are more soluble in water than is DMT base.

DMT can be distinguished from tylosin and other known tylosin factors by paper and thin-layer chromatography. The approximate Rf and Rx values of DMT and various other tylosin factors are summarized in Tables 11 and 12. In Table 12 Rx value is the ratio of movement expressed relative to that of tylosin, which was given a value of 1.0. Bioautography with *Bacillus subtilis* was used for detection.

*Table 11*

Thin-Layer Chromatography of DMT[a]

| Compound | Rf Value | | |
|---|---|---|---|
| | A[b] | B | C |
| Tylosin | 0.53 | 0.53 | 0.67 |
| DMT | 0.45 | 0.52 | 0.61 |

*Table 11* (continued)

Thin-Layer Chromatography of DMT[a]

| Compound | Rf Value | | |
|---|---|---|---|
| | A[b] | B | C |
| Desmycosin | 0.47 | 0.24 | 0.17 |
| Macrocin | 0.23 | 0.49 | 0.60 |
| Relomycin | 0.34 | 0.51 | 0.63 |

[a] Medium: Merck, Darmstadt – Silica Gel 60
[b] Solvent: A = ethyl acetate:diethylamine (96:4)
B = acetone:ethanol (2:1)
C = chloroform:methanol (3:1)

*Table 12*

Paper Chromatography of DMT

| Compound | Rx | |
|---|---|---|
| | D[b] | E |
| Tylosin | 1.0 | 1.0 |
| DMT | 0.76 | 0.95 |
| Desmycosin | 0.22 | 0.83 |
| Macrocin | 0.43 | 0.87 |
| Relomycin | 0.63 | 1.0 |

[a] Paper: Whatman No. 1 treated with 0.75 M $KH_2PO_4$ buffer at pH 4.0 and dried.
[b] Solvent: D = ethyl acetate saturated with water
E = n-butanol saturated with water.

*Example 3:*

*Preparation of OMT*

DMT, prepared as described in Example 2, was dissolved in dilute hydrochloric acid solution (HCl added to water until the pH of the solution was 1.8). The resulting solution was allowed to stand for 24 hours at room temperature and then adjusted to pH 9.0 by the addition of sodium hydroxide. This basic solution was extracted with ethyl acetate, dichloromethane or chloroform. The extract was dried under vacuum to give OMT.

*Example 4:*

*Preparation of OMT*

DMT (500 g), prepared as described in Example 2, was ground up with a mortar and pestle and added to deionized water (1.5 l). The pH was maintained between 3.5 and 7.5 by addition of sulfuric acid (20%), as necessary. After 30 min all DMT had dissolved. The pH of the solution was then adjusted to approximately 1.4 and allowed to stand at 22°C for 42 hours. After conventional work-up there was obtained 366 g of OMT the identity of which was confirmed by comparison of its TLC with a known authentic sample derived using the process described in U.S. Patent No. 3,459,853.

*Example 5:*

*Preparation of Dihydro-DMT*

DMT (50 mg), prepared as described in Example 2, was dissolved in an aqueous isopropyl alcohol solution (approximately 40%; 25 ml). Sodium borohydride (20 mg) was dissolved in a 30% aqueous isopropyl alcohol solution (10 ml). The $NaBH_4$ solution (1 ml) was added to the solution containing the DMT. The resulting mixture was stirred for 5 min, adjusted to pH 7.5 with phosphoric acid, and concentrated under vacuum to remove the isopropyl alcohol. Water was then added to the resulting aqueous concentrate to give a volume of 25 ml, whereupon chloroform (50 ml) was added. The pH of the aqueous phase was adjusted to 7.5. After extraction, the chloroform was separated and evaporated to dryness under vacuum to give dihydro-DMT.

*Example 6:*

*Preparation of Dihydro-OMT*

Dihydro-DMT, prepared as described in Example 5, was treated in the manner described in Example 3 to give dihydro-OMT.

*Example 7:*

*Alternative Preparation of OMT*

OMT was prepared from DMT by treating the DMT in the fermentation broth in which it was produced with mild acid as described in Example 3. Isolation of the OMT was accomplished by a procedure similar to that described for DMT in Example 2.

*Example 8:*

*2'-O-Acetyl-DMT*

DMT (10 g; 13.5 mmol) was dissolved in acetone (260 ml) and treated with acetic acid anhydride (1.6 ml; 15.7 mmol) dropwise with stirring at room temperature. After stirring overnight (18 hours), solvent was evaporated under reduced pressure. The residue was dissolved in ethyl acetate (200 ml) and extracted with saturated $NaHCO_3$ solution (2×200 ml). The organic solution was dried ($Na_2SO_4$), filtered and evaporated. The residue was dissolved in a small volume of ethyl acetate, loaded on a silica gel column (Waters Prep 500) and eluted with ethyl acetate (4 l). Fractions containing the desired product were identified by TLC analysis, combined and evaporated to dryness, yielding 6.5 g (61%) of 2'-O-acetyl-DMT.

*Example 9:*

*2'-O-Propionyl-DMT*

In an analogous manner, DMT (6.0 g, 8.1 mmol) in acetone (120 ml) was treated with propionic acid anhydride (1.2 ml, 9.2 mmol). After workup and chromatography, 3.7 g (57%) of 2'-O-propionyl-DMT was isolated.

*Example 10:*

*2',23-Di-O-Acetyl-DMT*

DMT (3 g, 4.05 mmol) was dissolved in methylene chloride (90 ml) and pyridine (7.8 ml) and treated with 1.4 ml (13.7 mmol) acetic acid anhydride dropwise with stirring at room temperature. After stirring overnight (17 hours), the solution was diluted with toluene (15 ml) and the solvents evaporated under reduced pressure. The residue was dissolved in a small volume of ethyl acetate, loaded on a silica gel column (Waters Prep 500) and eluted with ethyl acetate (4 l), yielding 2.1 g of crude product. This material was dissolved in a minimum volume of toluene, loaded onto a flash-chromatography column of silica gel (300 ml) and eluted with (a) 3:1 toluene-ethyl acetate (300 ml) (b) 5:4 toluene-ethyl acetate (600 ml) and (c) 1:1 toluene-ethyl acetate (1000 ml). Fractions containing the desired product were located by TLC analysis, combined and evaporated to dryness to yield 1.7 g (64%) of 2', 23-di-O-acetyl-DMT.

*Example 11:*

*2',23-Di-O-Propionyl-DMT*

DMT (3 g; 4.05 mmol) was dissolved in methylene chloride (90 ml) and pyridine (7.8 ml) and treated with 1.8 ml (13.8 mmol) propionic acid anhydride dropwise with stirring at room temperature. After stirring overnight, toluene (15 ml) was added and the solution evaporated to dryness under reduced pressure. The residue was dissolved in toluene (20 ml), loaded onto a flash-chromatography column of silica gel (E. Merck 60, 300 ml) and eluted with (a) 3:1 toluene-ethyl acetate (300 ml), (b) 5:4 toluene-ethyl acetate (300 ml) and (c) 1:1 toluene-ethyl acetate (1000 ml). 2.5 g (72%) of 2',23-di-O-propionyl-DMT was obtained.

*Example 12:*

*2'-O-Acetyl-23-O-Propionyl-DMT*

2'-O-Acetyl-DMT (6 g; 7.7 mmol) was dissolved in methylene chloride (180 ml) and pyridine (15 ml) and treated with 1.2 ml (9.2 mmol) propionic acid anhydride dropwise with stirring at room temperature. After stirring overnight (17 hours), the solution was diluted with toluene (300 ml) and evaporated to dryness under reduced pressure. The residue was dissolved in toluene and extracted with saturated $NaHCO_3$ solution; the toluene layer was dried ($Na_2SO_4$), filtered and evaporated. The residue was dissolved in a small volume of toluene, loaded onto a flash-chromatography column of silica gel (300 ml) packed in 1:1 toluene-ethyl acetate, and eluted with 1:1 toluene-ethyl acetate (1 l). Fractions containing the desired product were identified by TLC analysis, combined and evaporated under reduced pressure to yield 4.5 gm (70%) of 2'-O-acetyl-23-O-propionyl-DMT.

*Example 13:*

*2'-O-Propionyl-23-O-Acetyl-DMT*

In a similar manner, 2'-O-propionyl-DMT (1.5 g; 2 mmol) in methylene chloride (45 ml) and pyridine (3.9 ml) was treated with 0.3 ml (2.9 mmol) acetic acid anhydride. After chromatography on silica gel (Waters Prep 500), 0.81 gm (51%) of 2'-O-propionyl-23-O-acetyl-DMT was isolated.

*Example 14:*

*2'-O-Acetyl-23-O-Phenylacetyl-DMT*

2'-O-Acetyl-DMT (2.75 g; 3.5 mmol) was dissolved in methylene chloride (75 ml) and pyridine (0.8 ml) and treated with a solution of 0.56 ml (3.5 mmol) phenylacetyl chloride in methylene chloride (13 ml) dropwise with stirring at room temperature. After 1.5 hours, it was treated with additional phenylacetyl chloride (0.56 ml) in methylene chloride (13 ml). After another 1.5 hours, starting material had been consumed (TLC analysis). The solution was then evaporated to dryness under reduced pressure and the residue dissolved in methylene chloride and extracted with saturated $NaHCO_3$ solution. The organic layer was dried ($Na_2SO_4$), filtered and evaporated to dryness. The residue was dissolved in toluene and chromatographed on a silica gel column (Waters Prep 500). Elution was conducted with a linear gradient of 1:1 toluene-ethyl acetate (4 l) and ethyl acetate (4 l). Fractions containing the desired product were combined and evaporated to yield 1.1 g (35%) of 2'-O-acetyl-23-O-phenylacetyl-DMT. From earlier fractions was also obtained 0.86 gm (24%) of 2'-O-acetyl-23,4''-di-O-phenylacetyl-DMT.

*Example 15:*

*23-O-Propionyl-DMT*

2'-O-Acetyl-23-O-propionyl-DMT (1.6 g; 1.9 mmol) was dissolved in 95% MeOH (80 ml) and stirred at room temperature for 42 hours. The solution was evaporated to dryness under reduced pressure. The residue was dissolved in a small volume of toluene, loaded onto a flash-chromatography column of silica gel (E. Merck 60, 200 ml) and eluted with 1:1 toluene-ethyl acetate (2 l). Fractions containing the desired product were identified by TLC analysis, combined and evaporated under reduced pressure to yield 1.2 g (79%) of 23-O-propionyl-DMT.

*Example 16:*

*23-O-Phenylacetyl-DMT*

2'-O-Acetyl-23-O-phenylacetyl-DMT (0.6 g; 0.67 mmol) was dissolved in 80% aqueous methanol (50 ml) and gently refluxed for 4.5 hours. The solution was cooled to room temperature and evaporated to dryness under reduced pressure to yield 0.39 gm (68%) 23-O-phenylacetyl-DMT.

*Example 17:*

*23,4''-Di-O-Phenylacetyl-DMT*

2'-O-Acetyl-23,4''-di-O-phenylacetyl-DMT (0.36 g; 0.36 mmol) was dissolved in 80% aqueous methanol (30 ml) and refluxed for 4.5 hours. The solution was cooled to room

temperature and evaporated to dryness under reduced pressure to yield 0.29 g (84%) of 23,4"-di-O-phenylacetyl-DMT.

*Example 18:*

*2'-O-Acetyl-3,23,4"-Tri-O-Propionyl-DMT*

2'-O-acetyl-DMT (2 g; 2.6 mmol) was dissolved in acetone (40 ml) and pyridine (8 ml) and treated with a solution of 40 ml propionic acid anhydride in acetone (20 ml) dropwise with stirring at room temperature. After stirring for 5 days, the mixture was evaporated under reduced pressure. The residual oil was dissolved in ethyl acetate and extracted with saturated $NaHCO_3$ solution. The organic layer was dried ($Na_2SO_4$), filtered and evaporated. The residue was dissolved in a small volume of toluene and chromatographed on silica gel (Waters Prep 500). The column was eluted with a linear gradient of toluene (4 l) and ethyl acetate (4 l). Fractions containing the desired product were combined and evaporated to yield 1.4 g (57%) of 2'-O-acetyl-3,23,4"-tri-O-propionyl-DMT.

*Example 19:*

*3,23,4"-Tri-O-Propionyl-DMT*

2'-O-Acetyl-3,23,4"-tri-O-propionyl-DMT (0.7 g; 0.74 mmol) was dissolved in 80% aqueous methanol (55 ml) and refluxed for 5 hours. The solution was cooled and then evaporated to aqueous under reduced pressure. The product was extracted into methylene chloride and the organic layer was separated, dried ($Na_2SO_4$), filtered and evaporated under reduced pressure to dryness to yield 0.38 gm (56%) of 3,23,4"-tri-O-propionyl-DMT.

*Example 20:*

*2',4",23-Tri-O-Acetyl-DMT*

DMT (10.0 g; 13.5 mmol) was dissolved in pyridine (150 ml) and treated with 5.8 ml (60.7 mmol) acetic anhydride with stirring at room temperature. After stirring overnight, solvent was evaporated under reduced pressure. The residual oil was dissolved in dichloromethane and extracted with saturated $NaHCO_3$ solution; the organic layer was separated, dried ($Na_2SO_4$), filtered and evaporated under reduced pressure. The residual material was chromatographed on a silica gel column (Waters Prep 500), eluting with a linear gradient of 3:1 toluene-ethyl acetate (4 l) and ethyl acetate (4 l). Fractions containing the desired product were identified by TLC analysis, combined and evaporated under reduced pressure to yield 4.5 g of 2',4",23-tri-O-acetyl-DMT.

*Example 21:*

*2'-O-Acetyl-23-O-Phenoxyacetyl-DMT*

2'-O-Acetyl-DMT (2.75 g; 3.5 mmol) was dissolved in dichloromethane (75 ml) and pyridine (0.8 ml) and treated with a solution of 1.2 ml (8.8 mmol) phenoxyacetyl chloride in dichloromethane (25 ml) dropwise with stirring at room temperature. After 1 hour, the reaction mixture was poured into saturated $NaHCO_3$ solution (200 ml) and the organic layer was separated, dried ($Na_2SO_4$), filtered and evaporated under reduced pressure. The residual solid foam was loaded onto a flask-chromatography silica gel column and the products separated and eluted using 1:1 toluene-ethyl acetate as solvent. 1.5 g of 2'-O-acetyl-23-O-phenoxyacetyl-DMT was isolated along with 0.55 g of 2'-O-Acetyl-23,4"-di-O-phenoxyacetyl-DMT and 0.03 g of 2'-O-acetyl-3,23-di-O-phenoxyacetyl-DMT.

*Example 22:*

*2'-O-Acetyl-23-O-(p-chlorophenylacetyl)-DMT*

p-Chlorophenylacetic acid (4.3 g; 25 mmol) and 1-hydroxybenzotriazole (3.4 g; 25 mmol) were dissolved in THF (150 ml). The solution was cooled in an ice bath and treated with dicyclohexylcarbodiimide (5.2 g; 25.3 mmol). The reaction mixture was stirred at 0° C for 3 hours and then placed in a refrigerator overnight. The mixture was filtered and the filtrate evaporated under reduced pressure. The residue was dissolved in acetone (75 ml), filtered and treated with 10 g (12.8 mmol) 2'-O-acetyl-DMT and 0.87 g (12.8 mmol) imidazole. Acetone was added to bring the solution volume up to 125 ml and then 1.87 ml (12.8 mmol) triethylamine was added. After stirring for 20 hours at room temperature, solvent was evaporated under reduced pressure and the residue was loaded on a flask-chromatography silica gel column and eluted with a gradient of 4:1 toluene-ethyl acetate to ethyl acetate alone. 4.75 g of 2'-O-acetyl-23-O-(p-chlorophenylacetyl)-DMT was obtained.

*Example 23:*

*2'-O-Acetyl-4"-O-Propionyl-DMOT*

2'-O-Acetyl-DMOT (1.0 g; 1.3 mmol) was dissolved in pyridine (30 ml) and treated with 0.6 ml (4.6 mmol) propionic anhydride dropwise with stirring at room temperature. After 20 hours, an additional 3.0 ml (23 mmol) propionic anhydride was added and the reaction was stirred for an additional 26 hours at room temperature. The mixture was diluted with toluene (30 ml), and solvents were evaporated under reduced pressure. The residual oil was dissolved in dichloromethane (50 ml) and extracted with saturated $NaHCO_3$ solution; the organic layer was separated, dried ($Na_2SO_4$), filtered and evaporated. The residue was loaded on a silica gel column (Waters Prep 500) and eluted with a linear gradient of toluene (4 l) and ethyl acetate (4 l). 305 mg of 2'-O-acetyl-3,4"-di-O-propionyl-DMOT were eluted first followed by 286 mg of 2'-O-acetyl-4"-O-propionyl-DMOT.

**Claims** for the Contracting States BE, CH, LI, DE, FR, GB, IT, LU, NL, SE

1. A macrolide having the structural formula (I):

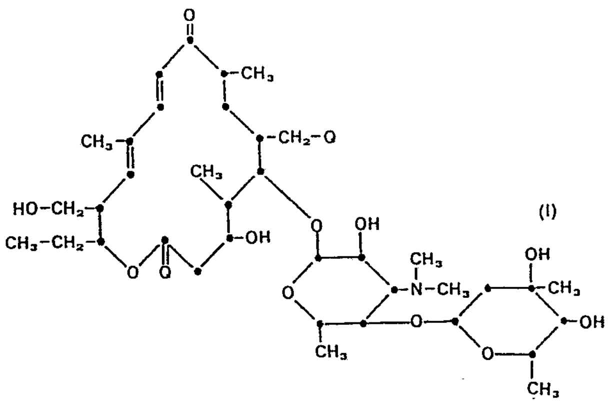

(I)

where Q is $-CH_2OH$ or $-CHO$;

or acyl esters thereof, derived from monocarboxylicacids having from 2 to 18 carbon atoms; or their acid-addition salts;
provided that when the 3-position of the molecule is occupied by an O-acetyl group, the 4''-position cannot be occupied by an O-isovaleryl group.

2. 23-Demycinosyltylosin.

3. 20-Dihydro-23-demycinosyltylosin.

4. A veterinary formulation which comprises as an active ingredient a macrolide of formula (I) as claimed in any one of claims 1 to 3, or a pharmaceutically-acceptable salt or acyl ester thereof, associated with at least one suitable inert carrier therefor.

5. A process for preparing a macrolide, salt or ester as claimed in any one of claims 1 to 3, by

(a) cultivating *Streptomyces fradiae* NRRL 12170 under submerged aerobic conditions until a substantial level of antibiotic activity is produced so as to obtain the macrolide, or

(b) reducing 23-demycinosyltylosin with a chemical reducing agent so as to form the macrolide wherein Q is $-CH_2OH$, and

(c) if it is desired to form the salt or ester, salifying or esterifying as appropriate.

6. A macrolide of formula (I) as claimed in any one of claims 1 to 3, or a pharmaceutically-acceptable salt or acyl ester thereof, for use in the chemotherapy of infections caused by microorganisms which are pathogenic to warmblooded animals.

7. A process for preparing 5-O-mycaminosyltylonolide (OMT) or 20-dihydro-5-O-mycaminosyltylonolide (dihydro-OMT) by acid hydrolysis of 23-demycinosyltylosin (DMT) or 20-dihydro-23-demycinosyltylosin (dihydro-DMT) at a pH of 4 or less.

8. *Streptomyces fradiae* NRRL 12170.

9. A culture medium containing *Streptomyces fradiae* NRRL 12170 and assimilable sources of carbon, nitrogen and inorganic salts.

**Claims** for the Contracting State: AT

1. A process for preparing a macrolide having the structural formula (I):

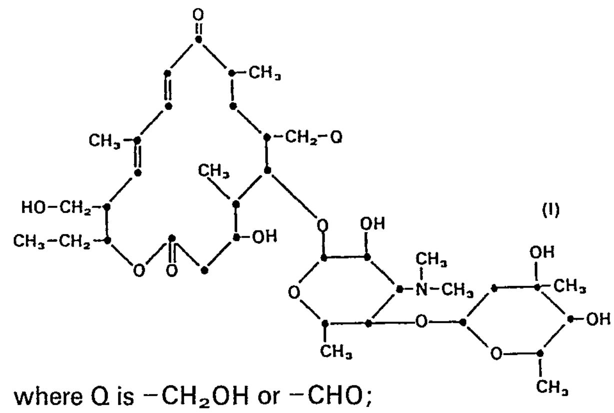

(I)

where Q is $-CH_2OH$ or $-CHO$;
or acyl esters thereof, derived from monocarboxylicacids having from 2 to 18 carbon atoms; or their acid-addition salts;
provided that when the 3-position of the molecule is occupied by an O-acetyl group, the 4''-position cannot be occupied by an O-isovaleryl group; by

(a) cultivating *Streptomyces fradiae* NRRL 12170 under submerged aerobic conditions until a substantial level of antibiotic activity is produced so as to obtain the macrolide, or

(b) reducing 23-demycinosyltylosin with a chemical reducing agent so as to form the macrolide wherein Q is $-CH_2OH$, and

(c) if it is desired to form the salt or ester, salifying or esterifying as appropriate.

2. A process according to claim 1 for preparing 23-demycinosyltylosin.

3. A process according to claim 1 for preparing 20-dihydro-23-demycinosyltylosin.

4. A process for preparing a veterinary formulation which comprises admixing an active ingredient comprising a macrolide of formula (I) as defined in claim 1, or a pharmaceutically-acceptable salt or acyl ester thereof with at least one suitable inert carrier.

5. As an antibiotic, a macrolide of formula (I) as defined in claim 1.

6. A process for preparing 5-O-mycaminosyltylonolide (OMT) or 20-dihydro-5-O-mycaminosyltylonolide (dihydro-OMT) comprising hydrolysing 23-demycinosyltylosin (DMT) or 20-dihydro-23-demycinosyltylosin (dehydro-DMT) at a pH of 4 on less.

**Patentansprüche** für die Vertragsstaaten BE, CH, LI, DE, FR, GB, IT, LU, NL, SE

1. Makrolid mit der Strukturformel (I)

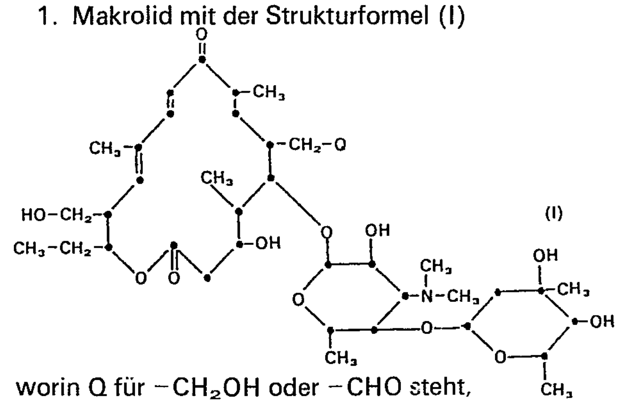

(I)

worin Q für $-CH_2OH$ oder $-CHO$ steht,

oder von Acylestern hiervon, die von Monocarbonsäuren mit 2 bis 18 Kohlenstoffatomen abgeleitet sind,
oder von Säureadditionssalzen hiervon,
mit der Massgabe, dass die Stellung 4" nicht durch eine O-Isovalerylgruppe besetzt sein kann, falls die Stellung 3 des Moleküls von einer O-Acetylgruppe besetzt ist.

2. 23-Demycinosyltylosin.

3. 20-Dihydro-23-demycinosyltylosin.

4. Veterinärformulierung, dadurch gekennzeichnet, dass sie ein Makrolid der Formel (I) gemäss einem der Ansprüche 1 bis 3 oder ein pharmazeutisch annehmbares Salz oder einen Acylester hiervon als Wirkstoff in Verbindung mit wenigstens einem geeigneten inerten Träger hierfür enthält.

5. Verfahren zur Herstellung eines Makrolids, Salzes oder Esters gemäss einem der Ansprüche 1 bis 3, dadurch gekennzeichnet, dass man

(a) *Streptomyces fradiae* NRRL 12170 unter submersen aeroben Bedingungen bis zur Bildung einer wesentlichen Menge an antibiotischer Aktivität züchtet und so das Makrolid erhält, oder

(b) 23-Demycinosyltylosin mit einem chemischen Reduktionsmittel reduziert und so das Makrolid bildet, worin Q für $-CH_2OH$ steht, und

(c) durch Ansäuerung oder Veresterung gewünschtenfalls das Salz oder den Ester bildet.

6. Verwendung eines Makrolids der Formel (I) gemäss einem der Ansprüche 1 bis 3 oder eines pharmazeutisch annehmbaren Salzes oder Acylesters hiervon zur Chemotherapie von durch Mikroorganismen, die Krankheitserreger für warmblütige Tiere sind, hervorgerufenen Infektionen.

7. Verfahren zur Herstellung von 5-O-Mycaminosyltylonolid (OMT) oder 20-Dihydro-5-O-mycaminosyltylonolid (Dyhydro-OMT), dadurch gekennzeichnet, dass man 23-Demycinosyltylosin (DMT) oder 20-Dihydro-23-demycinosyltylosin (Dihydro-DMT) bei einem pH von 4 oder niedriger umsetzt.

8. *Streptomyces fradiae* NRRL 12170.

9. Kulturmedium, dadurch gekennzeichnet, dass es *Streptomyces fradiae* NRRL 12170 und assimilierbare Quellen für Kohlenstoff, Stickstoff und anorganische Salze enthält.

**Patentansprüche** für den Vertragsstaat: AT

1. Verfahren zur Herstellung eines Makrolids der Strukturformel (I)

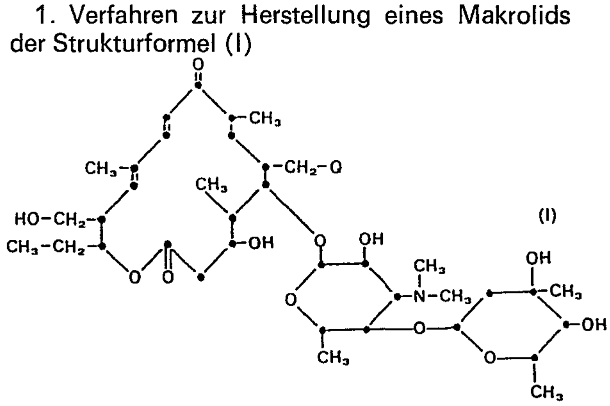

(I)

worin Q für $-CH_2OH$ oder $-CHO$ steht,
oder von Acylestern hiervon, die von Monocarbonsäuren mit 2 bis 18 Kohlenstoffatomen abgeleitet sind,
oder von Säureadditionssalzen hiervon,
mit der Massgabe, dass die Stellung 4" nicht durch eine O-Isovalerylgruppe besetzt sein kann, falls die Stellung 3 des Moleküls von einer O-Acetylgruppe besetzt ist, dadurch gekennzeichnet, dass man

(a) *Streptomyces fradiae* NRRL 12170 unter submersen aeroben Bedingungen bis zur Bildung einer wesentlichen Menge an antibiotischer Aktivität züchtet und so das Makrolid erhält, oder

(b) 23-Demycinosyltylosin mit einem chemischen Reduktionsmittel reduziert und so das Makrolid bildet, worin Q für $-CH_2OH$ steht, und

(c) durch Ansäuerung oder Veresterung gewünschtenfalls das Salz oder den Ester bildet.

2. Verfahren nach Anspruch 1, dadurch gekennzeichnet, dass man 23-Demycinosyltylosin herstellt.

3. Verfahren nach Anspruch 1, dadurch gekennzeichnet, dass man 20-Dihydro-23-demycinosyltylosin herstellt.

4. Verfahren zur Herstellung einer Veterinärformulierung, dadurch gekennzeichnet, dass man einen Wirkstoff aus einem Makrolid der Formel (I) gemäss Anspruch 1 oder einem pharmazeutisch annehmbaren Salz oder Acetylester hiervon mit wenigstens einem geeigneten inerten Träger vermischt.

5. Antibiotikum in Form eines Makrolids der Formel (I) gemäss Anspruch 1.

6. Verfahren zur Herstellung von 5-O-Mycaminosyltylonolid (OMT) oder 20-Dihydro-5-O-mycaminosyltylonolid (Dihydro-OMT), dadurch gekennzeichnet, dass man 23-Demycinosyltylosin (DMT) oder 20-Dihydro-23-demycinosyltylosin (Dihydro-DMT) bei einem pH von 4 oder niedriger umsetzt.

**Revendications** pour les Etats contractants BE, CH, LI, DE, FR, GB, IT, LU, NL, SE

1. Macrolide répondant à la formule structurale (I):

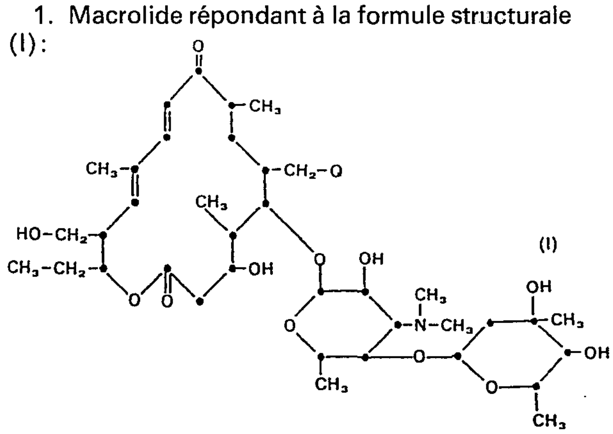

(I)

dans laquelle Q représente $-CH_2OH$ ou $-CHO$;
ou ses esters acyliques dérivant d'acides monocarboxyliques contenant 2 à 18 atomes de carbone;

ou ses sels d'addition d'acide; étant entendu que, lorsque la position 3 de la molécule est occupée par un groupe O-acétyle, la position 4" ne peut être occupée par un groupe O-isovaléryle.

2. La 23-démycinosyltylosine.

3. La 20-dihydro-23-démycinosyltylosine.

4. Formulation vétérinaire comprenant, comme ingrédient actif, un macrolide de formule (I) suivant l'une quelconque des revendications 1 à 3, un de ses esters acyliques ou de ses sels pharmaceutiquement acceptables, en association avec au moins un support inerte approprié pour ce composé.

5. Procédé de préparation d'un macrolide, d'un de ses sels ou d'un de ses esters suivant l'une quelconque des revendications 1 à 3, caractérisé en ce qu'il comprend les étapes qui consistent à:

(a) cultiver la souche *Streptomyces fradiae* NRRL 12170 dans des conditions aérobies submergées jusqu'à ce qu'on atteigne un important degré d'activité antibiotique pour obtenir le macrolide, ou

(b) réduire la 23-démycinosyltylosine avec un agent réducteur chimique afin de former le macrolide dans lequel Q représente $-CH_2OH$, et

(c) si l'on désire former le sel ou l'ester, salifier ou estérifier selon le cas.

6. Macrolide de formule (I) suivant l'une quelconque des revendications 1 à 3, un de ses esters acyliques ou un de ses sels pharmaceutiquement acceptables, en vue de l'utiliser dans la chimiothérapie des infections provoquées par les micro-organismes qui sont pathogènes vis-à-vis des animaux à sang chaud.

7. Procédé de préparation du 5-O-mycaminosyltylonolide (OMT) ou du 20-dihydro-5-O-mycaminosyltylonolide (dihydro-OMT) par hydrolyse acide de la 23-démycinosyltylosine (DMT) ou de la 20-dihydro-23-démycinosyltylosine (dihydro-DMT) à un pH de 4 ou moins.

8. *Streptomyces fradiae* NRRL 12170.

9. Milieu de culture contenant la souche *Streptomyces fradiae* NRRL 12170, ainsi que des sources assimilables de carbone, d'azote et de sels inorganiques.

**Revendications** pour l'Etat contractant: AT

1. Procédé de préparation d'un macrolide répondant à la formule structurale (I):

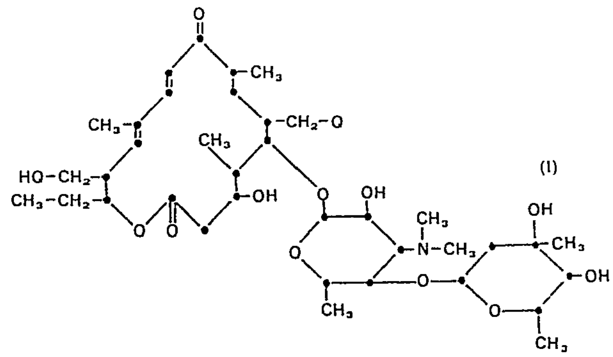

(I)

dans laquelle Q représente $-CH_2OH$ ou $-CHO$; ou de ses esters acyliques dérivant d'acides monocarboxyliques contenant 2 à 18 atomes de carbone; ou encore de ses sels d'addition d'acide; étant entendu que, lorsque la position 3 de la molécule est occupée par un groupe O-acétyle, la position 4" ne peut être occupée par un groupe O-isovaléryle, caractérisé en ce qu'il comprend les étapes qui consistent à:

(a) cultiver la souche *Streptomyces fradiae* NRRL 12170 dans des conditions aérobies submergées jusqu'à ce qu'on atteigne un important degré d'activité antibiotique pour obtenir le macrolide, ou

(b) réduire la 23-démycinosyltylosine avec un agent réducteur chimique afin de former le macrolide dans lequel Q représente $-CH_2OH$, et

(c) si l'on désire former le sel ou l'ester, salifier ou estérifier selon le cas.

2. Procédé suivant la revendication 1 pour la préparation de la 23-démycinosyltylosine.

3. Procédé suivant la revendication 1 pour la préparation de la 20-dihydro-23-démycinosyltylosine.

4. Procédé de préparation d'une formulation vétérinaire, caractérisé en ce qu'il consiste à mélanger un ingrédient actif comprenant un macrolide de formule (I) telle que définie dans la revendication 1, ou un de ses esters acyliques ou de ses sels pharmaceutiquement acceptables avec au moins un support inerte approprié.

5. En tant qu'antibiotique, un macrolide de formule (I) telle que définie dans la revendication 1.

6. Procédé de préparation du 5-O-mycaminosyltylonolide (OMT) ou du 20-dihydro-5-O-mycaminosyltylonolide (dihydro-OMT), caractérisé en ce qu'il consiste à hydrolyser la 23-démycinosyltylosine (DMT) ou la 20-dihydro-23-démycinosyltylosine (dihydro-DMT) à un pH de 4 ou moins.

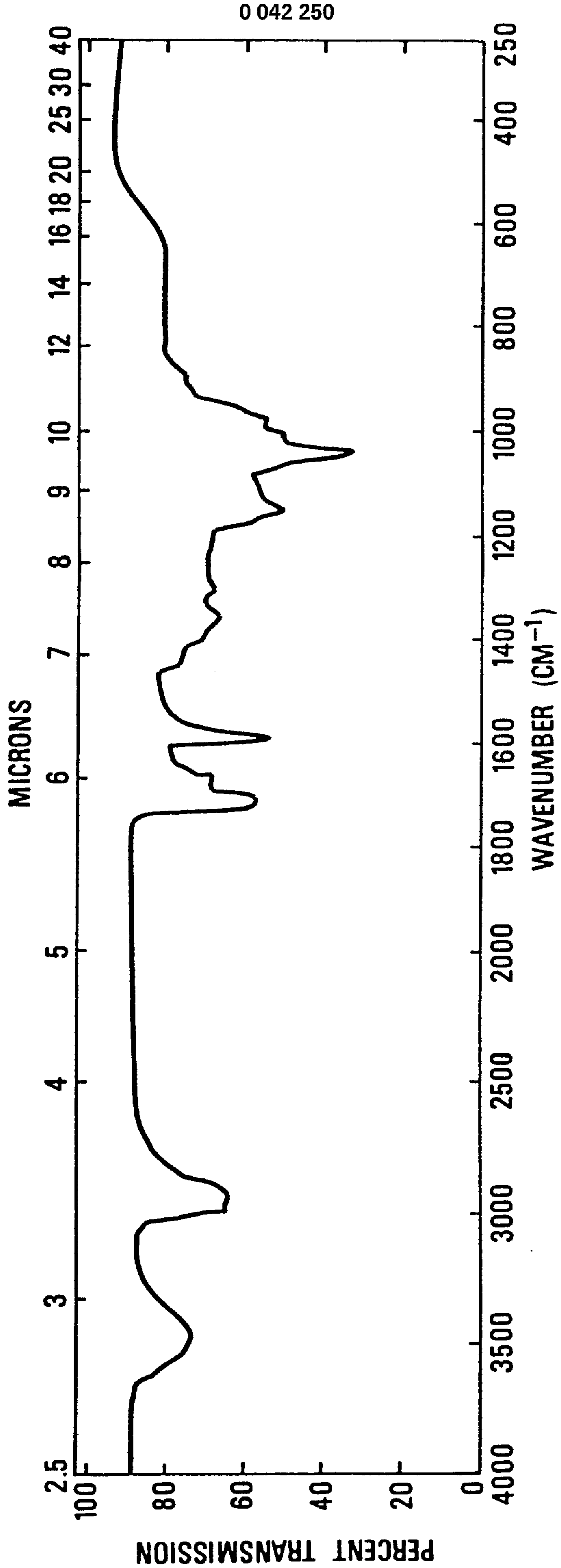
MICRONS
2.5
3
4
5
6
7
8
9
10
12
14
16 18 20
25 30 40
WAVENUMBER (CM$^{-1}$)
4000
3500
3000
2500
2000
1800
1600
1400
1200
1000
800
600
400
250
PERCENT TRANSMISSION
100
80
60
40
20
0